# EUROPEAN PATENT SPECIFICATION

(11) **EP 2 070 530 B1**
(45) Date of publication and mention of the grant of the patent: **17.02.2016**
(21) Application number: 09156339.5
(22) Date of filing: 13.05.2005
(51) Int. Cl.: A61K 31/335, A61K 31/7048, A61K 31/365, A61P 1/12, A61K 45/06

(54) **Treatment of diseases associated with the use of antibiotics**
Behandlung von Krankheiten im Zusammenhang mit der Anwendung von Antibiotika
Traitement de maladies consecutives a un traitement antibiotique

(30) Priority: 14.05.2004 US 570697 P
(43) Date of publication of application: 17.06.2009
(62) Divisional of application: 05748007.1
(73) Proprietor: Merck Sharp & Dohme Corp., Rahway, NJ 07065 (US)
(72) Inventor: Shue, Youe-Kong, Carlsbad, CA 92009 (US); Babakhani, Farah Kondori, San Diego, CA 92131 (US); Okumu, Franklin W., Oakland, CA 94619 (US); Sears, Pamela Suzanne, San Diego, CA 92117 (US); Miller-Shangle, Starr Louise, San Diego, CA 92117 (US); Walsh, Robert Brian, Davis, CA 95616 (US)
(74) Representative: Jaap, David Robert

(56) References cited:
- WO-A-2004/014295
- US-A- 5 583 115
- SWANSON R N ET AL: "In vitro and in vivo evaluation of tiacumicins B and C against Clostridium difficile" ANTIMICROBIAL AGENTS AND CHEMOTHERAPY, AMERICAN SOCIETY FOR MICROBIOLOGY, WASHINGTON, DC, US, vol. 35, no. 6, 1 June 1991 (1991-06-01), pages 1108-1111, XP002993697 ISSN: 0066-4804

## Description

### FIELD OF THE INVENTION

This invention relates to the treatment of diseases due to *C. difficile.*

### BACKGROUND OF THE INVENTION

Antibiotic-associated diarrhea (AAD) diseases are caused by toxin producing strains of *Clostridium difficile (C. difficile), Staphylococcus aureus (S. aureus)* including MRSA and *Clostridium perfringens (C. perfringens).* AAD represents a major economic burden to the healthcare system that is conservatively estimated at $3-6 billion per year in excess hospital costs in the U.S. alone.

Vancomycin resistant enterococci, which most commonly results in intestinal colonization, has also emerged as a major nosocomial pathogen associated with increased health care cost and mortality. VRE can appear as confection in patients infected with *C. difficile,* or more commonly cause infection in certain high risk patients such as haematology and oncology patients, patients in intensive care units and patients receiving solid organ transplants.

Methicillin-resistant Staphylococci, such as methicillin-resistant *Staphylococcus aureus* (MRSA), are increasing in prevalence in both the hospital and community settings. Staphylococci are found on the skin and within the digestive and respiratory tracts but can infect open wounds and burns and can progress to serious systemic infection. The emergence of multi-drug resistant Staphylococci, especially, in the hospital where antibiotic use is frequent and this selective pressure for drug-resistant organism is high, has proven a challenge for treating these patients. The presence of MRSA on the skin of patients and health care workers promotes transmission of the multi-drug resistant organisms.

Similar diseases, including but not limited to clostridial enterocolitis, neonatal diarrhea, antibiotic-associated enterocolitis, sporadic enterocolitis, and nosocomial enterocolitis are also significant problems in some animal species.

AAD is a significant problem in hospitals and long-term care facilities and in the community. *C. difficile* is the leading cause of AAD in the hospital setting, accounting for approximately 20% of cases of AAD and the majority of cases of antibiotic-associated colitis (AAC). The rising incidence of *Clostridium difficile* associated diarrhea (CDAD) has been attributed to the frequent prescription of broad-spectrum antibiotics to hospitalized patients.

The most serious form of the disease is pseudomembranous colitis (PMC), which is manifested histologically by colitis with mucosal plaques, and clinically by severe diarrhea, abdominal cramps, and systemic toxicity. The overall mortality rate from CDAD is low, but is much greater in patients who develop severe colitis or systemic toxicity. A recent study has shown that even when death is not directly attributable to *C*. *difficile,* the rate of mortality in CDAD patients as compared to case-matched controls is much greater.

Diarrhea and colitis are caused by the elaboration of one or more *C*. *difficile* toxins. The organism proliferates in the colon in patients who have been given broad-spectrum antibiotics or, less commonly, cancer chemotherapy. CDAD is diagnosed in approximately 20% of hospitalized patients who develop diarrhea after treatment with such agents.

There are currently two dominant therapies for CDAD: vancomycin and metronidazole. Vancomycin is not recommended for first-line treatment of CDAD mainly because it is the only antibiotic active against some serious life-threatening multi-drug resistant bacteria. Therefore, in an effort to minimize the emergence of vancomycin-resistant *Enterococcus* (VRE) or vancomycin-resistant *Staphylococcus aureus* (VRSA), the medical community discourages the use of this drug except when absolutely necessary.

Metronidazole is recommended as initial therapy out of concern for the promotion and selection of vancomycin resistant gut flora, especially enterococci. Despite reports that the frequency of *C*. *difficile* resistance may be >6% in some countries, metronidazole remains nearly as effective as vancomycin, is considerably less expensive, and can be used either orally or intraveneously. Metronidazole is associated with significant adverse effects including nausea, neuropathy, leukopenia, seizures, and a toxic reaction to alcohol. Furthermore, it is not safe for use in children or pregnant women. Clinical recurrence occurs in up to 20% of cases after treatment with either vancomycin or metronidazole. Therapy with metronidazole has been reported to be an important risk factor for VRE colonization and infection. In addition, the current treatment regime is rather cumbersome, requiring up to 500 mg *qid* for 10 to 14 days. Thus, there is a need for better treatment for cases of CDAD as well as for cases of other AAD and AAC.

Compound 1 contains Tiacumicin B, which belongs to a member of a family of 18-membered macrocycles, Tiacumicins. Tiacumicins are produced by bacteria, including *Dactylosporangium aurantiacum* subspecies *hamdenensis,* which may be obtained from the ARS Patent Collection of the Northern Regional Research Center, United States Department of Agriculture, 1815 North University Street, Peoria, IL 61604, accession number NRRL 18085. The characteristics of strain AB 718C-41 are given in J. Antibiotics, 1987, 567-574 and US Patent No. 4,918,174.

Tiacumicins, specifically Tiacumicin B, show activity against a variety of bacterial pathogens and in particular against *Clostridium difficile,* a Gram-positive bacterium (Antimicrob. Agents Chemother. 1991, 1108-1111). *Clostridium difficile* is an anaerobic spore-forming bacterium that causes an infection of the bowel. Diarrhea is the most common symptom but abdominal pain and fever may also occur. *Clostridium difficile* is a major causative agent of colitis (inflammation of the colon) and diarrhea that may occur following antibiotic intake. This bacterium is primarily acquired in hospitals and chronic care facilities. Because Tiacumicin B shows promising activity against *C*. *difficile,* it is expected to be useful in the treatment of bacterial infections, especially those of the gastrointestinal tract, in mammals. Examples of such treatments include but are not limited to treatment of colitis and treatment of irritable bowel syndrome. Tiacumicins may also find use for the treatment of gastrointestinal cancers.

### SUMMARY OF THE INVENTION

The invention relates to the use according to claim 1. Further embodiments of the invention are set out in claims 2 to 15.

Compound I is typically administered in an amount between 10 mg and 1 g, although higher or lower doses may be required. Administration may be daily (e.g., one to four times daily) or may be less frequent (e.g., once every other day or once or twice weekly). In a desired embodiment, Compound I is administered in an amount between 50 and 400 mg once or twice daily. While the duration of Compound I therapy is determined on a case-by-case basis, typically administration is for three to fifteen days. Treatment durations shorter than standard therapies may be warranted with Compound I. Oral administration is preferred.

The Compound I administration may be performed in conjunction with other therapies. For example, the patient may also receive a biotherapy (e.g., (*Saccharomyces boulardii*), or oral yoguart *(e.g., Lactobacillus preparations), or Lactobacillus GG,* or an immunotherapy (e.g., human immune globulin, *C*. *difficile* toxoid vaccine), or a second antibiotic (e.g., vancomycin, bacitracin, or metronidazole). Compound I may be co-formulated with any of the foregoing, or may be administered separately.

The treatment of the present invention allows for the effective treatment of diarrhea diseases associated with enterotoxigenic strains of *C. difficile* without compromising systemic antibiotics and without increasing vancomycin resistant enterococci (VRE) in the gut. The present invention also reduces the presence of VRE in the gut. Other features and advantages will be apparent from the description.

### BRIEF DESCRIPTION OF THE DRAWINGS

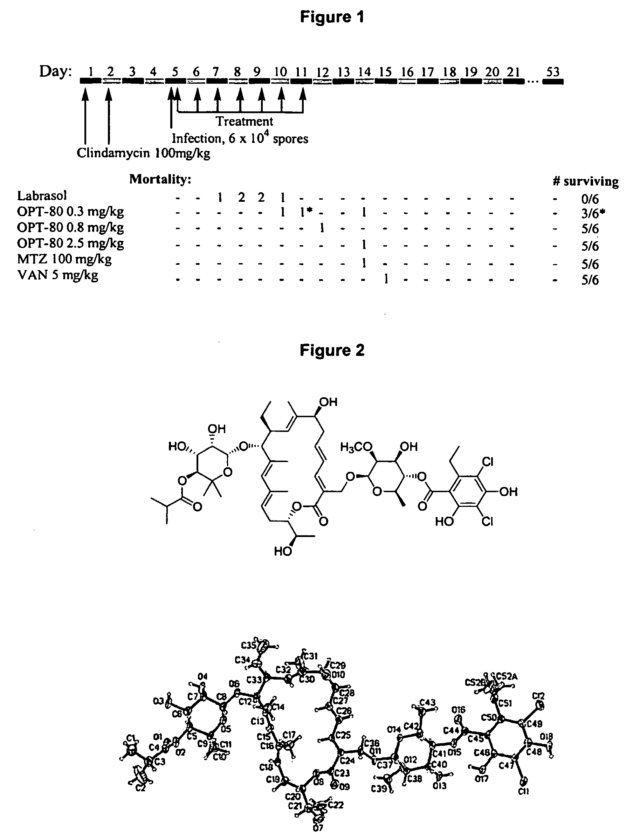

Figure. 1 is a graph showing the comparative efficiency of Compound I, vancomycin or metronidazole on clindamycin-induced CDAD in Syrian hamsters. Figure. 2 ORTEP Diagram of the Structure of the main component of Compound I.

### DETAILED DESCRIPTION OF THE INVENTION

### Abbreviations and Definitions

AAC = antibiotic-associated colitis
AAD = antibiotic-associated diarrhea
ATCC = American Type Culture Collection
13 C = carbon 13
CDAD = *Clostridium difficile*-associated diarrhea
EC = emphysematous cholecystitis
ED₅₀ = effective dose 50
HPLC = high performance liquid chromatography
IR = infrared spectroscopy
LLOQ = lower limit of quantification
MIC = minimum inhibitory concentration
MIC₅₀ = minimum inhibitory concentration to inhibit 50% of bacterial strains tested
MIC₉₀ = minimum inhibitory concentration to inhibit 90% of bacterial strains tested MRSA = methicillin-resistant *Staphylococcus aureus*
NCCLS = National Commmittee for Clinical Laboratory Standards NMR = nuclear magnetic resonance
ORTEP = Oak Ridge Thermal Ellipsoid Plot
PMC = pseudomembranous colitis
UV-vis = ultraviolet/visual
VRE = vancomycin-resistant enterococci
VRSA = vancomycin-resistant *Staphylococcus aureus*

The term "antibiotic-associated condition" refers to a condition resulting when antibiotic therapy disturbs the balance of the microbial flora of the gut, allowing pathogenic organisms such as enterotoxin producing strains of *C. difficile, S. aureus* and *C. perfringens* to flourish. These organisms can cause diarrhea, pseudomembranous colitis, and colitis and are manifested by diarrhea, urgency, abdominal cramps, tenesmus, and fever among other symptoms. Diarrhea, when severe, causes dehydration and the medical complications associated with dehydration.

The term "autism" refers to a spectrum of complex developmental disorder of childhood, characterized by pervasive impairments in social interaction, deficits in verbal and nonverbal communication, and repetitive behavioral patterns, developing within the first 3 years of life.

The term "Compound I" refers to a preparation containing approximately 90% (with respect to the whole antibiotic substance, by HPLC assay) of Tiacumicin B with a range of between 80-100%. The remaining portions consist essentially of small amounts of Tiacumicin B related compounds. Preparations of this type are described in detail in PCT application PCT/US03/21977, having an international publication number of WO 2004/014295 A2. However, Compound I intended exclusively for use in non-humans may contain less than 80% of Tiacumicin B (with respect to the whole antibiotic substance, by HPLC assay).

The term "enteric coating" refers to a coating that encapsulates a pharmaceutical composition, and prevents release and degradation from occurring in the stomach, while dissolving readily in the mildly acidic or neutral pH environment of the small intestine. Other similar coatings include time-dependent, pH-dependent, and enzymatic erosion of polymer matrix coatings.

The term "excipient" refers to an inert substance added to a pharmacological composition to further facilitate administration of a compound. Examples of excipients include but are not limited to, calcium carbonate, calcium phosphate, various sugars and types of starch, cellulose derivatives, gelatin, vegetable oils and polyethylene glycols.

The term "halogen" includes F, Cl, Br and I.

The term "macrocycles" refers to organic molecules with large ring structures usually containing over 10 atoms.

The term "18-membered macrocycles" refers to organic molecules with ring structures containing 18 atoms.

The term "membered ring" can embrace any cyclic structure, including carbocycles and heterocycles as described above. The term "membered" is meant to denote the number of skeletal atoms that constitute the ring. Thus, for example, pyridine, pyran and thiopyran are 6 membered rings and pyrrole, furan, and thiophene are 5 membered rings.

The term "MIC" or "minimum inhibitory concentration" refers to the lowest concentration of an antibiotic that is needed to inhibit growth of a bacterial isolate in vitro. A common method for determining the MIC of an antibiotic is to prepare several tubes containing serial dilutions of the antibiotic, that are then inoculated with the bacterial isolate of interest. The MIC of an antibiotic can be determined from the tube with the lowest concentration that shows no turbidity (no growth).

The term "MIC₅₀" refers to the lowest concentration of antibiotic required to inhibit the growth of 50% of the bacterial strains tested within a given bacterial species.

The term "MIC₉₀" refers to the lowest concentration of antibiotic required to inhibit the growth of 90% of the bacterial strains tested within a given bacterial species.

The term "ORTEP" refers to the Oak Ridge Thermal Ellipsoid Plot computer program, written in Fortran, for drawing crystal structure illustrations. Ball-and-stick type illustrations of a quality suitable for publication are produced with either spheres or thermal-motion probability ellipsoids, derived from anisotropic temperature factor parameters, on the atomic sites. The program also produces stereoscopic pairs of illustrations which aid in the visualization of complex arrangements of atoms and their correlated thermal motion patterns.

The term "patient" refers to a human or animal in need of medical treatment. For the purposes of this invention, human patients are typically institutionalized in a primary medical care facility such as a hospital or nursing home. However, treatment of a disease associated with the use of antibiotics or cancer chemotherapies or antiviral therapies can occur on an outpatient basis, upon discharge from a primary care facility, or can be prescribed by a physician for home-care, not in association with a primary medical care facility. Animals in need of medical treatment are typically in the care of a veterinarian.

The term "pharmaceutically acceptable carrier" refers to a carrier or diluent that is pharmaceutically acceptable.

The term "pharmaceutically acceptable salts" refers to those derived from pharmaceutically acceptable inorganic and organic bases. Salts derived from appropriate bases include alkali metal (*e.g.,* sodium or potasium), alkaline earth metal (*e.g.,* magnesium), ammonium and N(C₁-C₄ alkyl)₄⁺ salts, and the like. Illustrative examples of some of these include sodium hydroxide, potassium hydroxide, choline hydroxide, sodium carbonate, and the like.

The term "pharmaceutical composition" refers to a mixture of one or more of the Tiacumicins described herein, or physiologically acceptable salts thereof, with other chemical components, such as physiologically acceptable carriers and/or excipients. The purpose of a pharmaceutical composition is to facilitate administration of a compound to an organism.

The term "physiologically acceptable carrier" refers to a carrier or diluent that does not cause significant irritation to an organism and does not abrogate the biological activity and properties of the administered compound.

The term "pseudomembranous colitis" or "enteritis" refers to the formation of pseudomembranous material (i.e., material composed of fibrin, mucous, necrotic epithelial cells and leukocytes) due to inflammation of the mucous membrane of both the small and large intestine.

The term "sugar" generally refers to mono-, di- or oligosaccharides. A saccharide may be substituted, for example, glucosamine, galactosamine, acetylglucose, acetylgalactose, N-acetylglucosamine, N-acetyl-galactosamine, galactosyl-N-acetylglucosamine, N-acetylneuraminic acid (sialic acid), etc., as well as sulfated and phosphorylated sugars. For the purposes of this definition, the saccharides are in their pyranose or furanose form.

The term "Tiacumicin" as used herein refers to a family of compounds all of which comprise the 18-membered macrocycle shown below in Formula I:

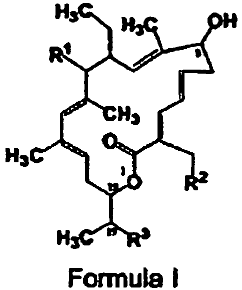

Formula I The term "Tiacumicin B" as used herein refers to the 18-membered macrocycle shown below in Formula II:

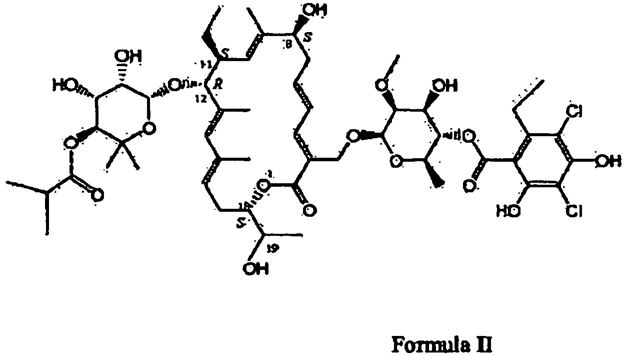

The present invention relates to the unexpected discovery that conditions associated with the use of antibiotics or cancer chemotherapies or antiviral therapies, such as diarrhea associated with *C. difficile* can be treated or prevented by the administration of an effective amount of Compound I in patients. The subject antibiotic-associated conditions include, but are not limited to, antibiotic-associated diarrhea, pseudomembranous colitis, colitis, blood stream infection prevention. This discovery may be particularly relevant in patients at risk for enterococci infections, including vancomycin resistant enterococci (VRE).

The present invention includes uses for the treatment and prevention of similar diseases and conditions, including infection caused by *Clostridium* spp. and which may or may not be associated with the use of antibiotics, in non-human species, including but not limited to horses and other equines, dogs, and cats.

The present invention also provides combination therapies for the treatment and prevention of the subject antibiotic associated conditions. By adding an effective amount of Compound I to the standard courses of broad-spectrum antibiotics, the treatments of the present invention prevent the growth of *C. difficile* and other bacteria known to cause antibiotic-associated diarrheal diseases. Antibiotics used in conjunction with Compound I in the combination therapies of the present invention include, but are not limited to, vancomycin, bacitracin, and metronidazole. Compound I may be co-formulated with any of the foregoing, or may be administered separately.

The present invention also provides combination therapies for the treatment and prevention of the subject antibiotic associated conditions by adding an effective amount of Compound I with a biotherapy. Biotherapies used in conjunction with Compound I of the present invention include, but are not limited to *Saccharomyces boulardii* and oral yoguart, *Lactobacillus preparations, or Lactobacillus GG.* Compound I may be co-formulated with any of the foregoing, or may be administered separately.

The present invention also provides combination therapies for the treatment and prevention of the subject antibiotic associated conditions by adding an effective amount of Compound I with an immunotherapy. Immunotherapies used in conjunction with Compound I of the present invention include, but are not limited to human immue globulin or *C. difficile* toxoid vaccine. Compound I may be co-formulated with any of the foregoing, or may be administered separately.

The present invention also contemplates compositions and methods for the treatment of symptoms associated with antibiotic associated conditions, which result when antibiotics allow certain bacteria such as toxigenic strains of *C. difficile,* to flourish in the gut An effective amount of Compound I could be combined with preparations to treat the dehydration resulting from chronic diarrhea, including, but not limited to, intravenous fluids or over-the-counter drinks containing electrolytes.

The present invention also contemplates compositions and methods for preventing blood stream infection, which result when antibiotics allow certain bacteria such as toxigenic strains of *C. difficile* to flourish in the gut.

### Clostridium difficile

*C. difficile* is a gram-positive anaerobic, spore-forming bacillus and is the pathogen that causes antibiotic associated diarrhea/colitis, and almost all cases of pseudomembranous colitis. These conditions develop as a result of the over growth of toxigenic *C. difficile* that produces one or more toxins, toxin A and toxin B in the colon. Toxin A is a potent enterotoxin and is believed to cause most of the gastrointestinal symptoms. Also, evidence indicated that toxins A and B act synergistically causing tissue damage. The combined effects of toxin A and toxin B initiate an inflammatory response in the colonic mucosa, once infection by *C. difficile* is established.

Symptomatically, the patient experiences abdominal cramps/pain, tenesmus, urgency, diarrhea (including bloody diarrhea) and fever among other symptoms. Progression of the disease results in full mucosal cell death and the appearance of pseudomembranes. Dilatation of colon, perforation, peritonitis, sepsis, and even death may result. It can appear when the normal bacterial flora in the colon is suppressed, e.g., after treatment with broad-spectrum antibacterial agents. The overuse of antibiotics, especially penicillin, ampicillin, clindamycin, and cephalosporins alter the normal intestinal flora and increase the risk of developing *C*. *difficile* infections, often endemic in hospitals and nursing home settings.

Antibiotic use is the major risk factor for CDAD. Also, age appears to be a risk factor as the majority of the cases appear in patients 65 or older. Other patients at risk include postoperative patients, patients undergoing chemotherapy, patients with bone marrow transplants, and patients with compromised immune system.These immunological conditions may include, but are not limited to, cancer,malnutrition, infection with human immunodeficiency virus, and connective tissue disorders (e.g., lupus erythematosus, Sjogren's Syndrome). Moreover, these patients are also at risk for VRE colonization and infection (Fry, Pharmanual: Emerging Pathogens and Implications for the Future (1999) pp. 50-75). Thus, these populations may also benefit from the methods of treatment and compositions described herein.

### Compound I

Compound 1 is a preparation containing approximately 90% (with respect to the whole antibiotic substance, by HPLC assay) of Tiacumicin B with a range of between 80-100%. The remaining portions consist essentially of small amounts of Tiacumicin B related compounds. Tiacumicins are a family of related compounds (Tiacumicin A-F) that contain the 18-membered macrocycles ring shown in Table 1.

Tiacumicins A-F have been characterized spectroscopically and by other physical methods. The chemical structures of Tiacumicins are based on spectroscopy: UV-vis, IR and ¹H and ¹³C NMR. Certain stereochemical features have been determined using 1D and 2D homonuclear and heteronuclear NMR experiments, see for example J. Antibiotics, 1987, 575-588. In the case of Tiacumicin B, the molecular structure was confirmed by X-ray diffraction (Figure 2). The X-ray crystal structure of Tiacumicin B was obtained from a colorless, parallelepiped-shaped crystal (0.08 x 0.14 x 0.22 mm) grown in methanol.

**Table 1. Tiacumicin A-F**

| | | | |
|---|---|---|---|
| 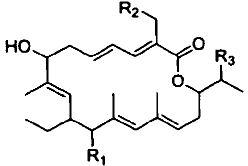 | | | |

| | Position^{a}:R₁ | R₂ | R₃ |
|---|---|---|---|
| A | 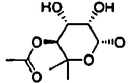 | 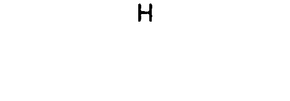 | H |
| B | 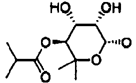 | 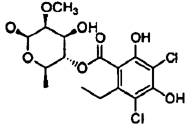 | OH |
| C | 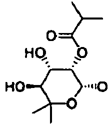 | 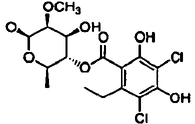 | OH |
| D | 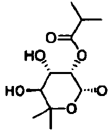 | 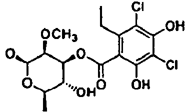 | OH |
| E | 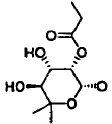 | 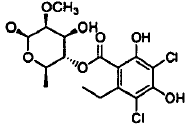 | OH |
| F | 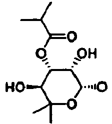 | 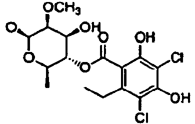 | OH |

### Dosages

Compound I is administered orally in an amount and for a duration sufficient to treat CDAD, pseudomembranous colitis, or other diseases associated with the use of antibiotics or cancer chemotherapies. Although the exact dosage of Compound I sufficient to treat a particular patient may differ, the dosage can be easily determined by a person of ordinary skill. Typically, the amount of Compound I that is administered is an amount that maintains the stool concentration of the antibiotic at least equal to the MIC for the target organism.

Preferably, the amount of Compound I that is administered maintains the stool concentration equivalent to two, three, four, or more times the MIC for the target organism. Thus, the particular treatment regimen may vary for each patient, dependent upon the species and resistance pattern of the identified gram-positive bacteria, and biological factors unique to each patient including the comorbidity, disease etiology, patient age (pediatric, adult, geriatric), and the nutritional and immune status.

The suggested oral dosage of Compound I is at least about 25, 50, 100, 200, 300, 400, or 500 mg/day up to as much as 600, 700, 800, 900, or 1000 mg/day for three to fifteen days. Compound I may be given daily (e.g., once, twice, three times, or four times daily) or less frequently (e.g., once every other day, or once or twice weekly). A particularly suitable dose is between 50 and 400 mg BID (twice daily). The antibiotic may be contained in any appropriate amount in any suitable carrier substance, and is generally present in an amount of 1-99% by weight of the total weight of the composition. The composition is provided in a dosage form that is suitable for oral administration and delivers a therapeutically effective amount of the antibiotic to the small and large intestine, as described below.

Compound I is available as granules for oral solution, provided, for example, in packets containing 100 mg of Compound I, along with pharmaceutically acceptable excipients (e.g., mannitol, hydroxypropyl methylcellulose, magnesium stearate). The contents of the packet can be reconstituted with approximately 15-30 mL of water, and the resulting solution either consumed directly, or further diluted with water, cranberry juice, apple juice, or 7-Up prior to drinking. After consumption, the drug may be followed with subsequent amounts of these beverages or with food (e.g., cracker, bread).

Compound I is also available as a tablet containing pharmaceutically 30 acceptable excipients that are generally regarded as safe. The tablet may be available as 25 mg, 50 mg, 100 mg, 200 mg or 400 mg strengths.

Alternatively, Compound I is also available as capsules containing pharmaceutically acceptable excipients that are generally regarded as safe. The capsule formulation may be available as 25 mg, 50 mg, 100 mg, 200 mg or 400 mg strengths.

The dosing regimen required to treat CDAD, pseudomembranous colitis, or other diseases associated with the use of antibiotics or cancer chemotherapies or antiviral therapies may be altered during the course of the therapy. For example, the patient can be monitored periodically or at regular intervals to measure the patient's bacterial load and dosage or frequency of antibiotic therapy can be adjusted accordingly. Compound I may be dosed for a duration shorter or similar to that of commonly used treatments.

### Pharmaceutical Formulations

Pharmaceutical compositions of Compound I, according to the invention may be formulated to release an antibiotic substantially immediately upon administration or at any predetermined time or time period after administration.

The latter types of compositions are generally known as modified release formulations, which include formulations that create a substantially constant concentration of the drug within the intestinal tract over an extended period of time, and formulations that have modified release characteristics based on temporal or environmental criteria as described in Modified-Release Drug Delivery Technology, ed. M. J. Rathbone, J. Hodgraft and M. S. Roberts. Marcel Dekker, Inc. New York.

Any oral biologically-acceptable dosage form, or combinations thereof, can be employed in the methods of the invention. Examples of such dosage forms include, without limitation, chewable tablets, quick dissolve tablets, effervescent tablets, reconstitutable powders, elixirs, liquids, suppositiory, creams, solutions, suspensions, emulsions, tablets, multi-layer tablets, bi-layer tablets, capsules, soft gelatin capsules, hard gelatin capsules, osmotic tablets, osmotic capsules, caplets, lozenges, chewable lozenges, beads, powders, granules, particles, microparticles, dispersible granules, ingestibles, infusions, health bars, confections, animal feeds, cereals, cereal coatings, foods, nutritive foods, functional foods and combinations thereof. The preparation of any of the above dosage forms is well known to persons of ordinary skill in the art. Additionally, the pharmaceutical formulations may be designed to provide either immediate or controlled release of the antibiotic upon reaching the target site. The selection of immediate or controlled release compositions depends upon a variety of factors including the species and antibiotic susceptibility of Gram-positive bacteria being treated and the bacteriostatic/bactericidal characteristics of the therapeutics. Methods well known in the art for making formulations are found, for example, in Remington: The Science and Practice of Pharmacy (20th ed.), ed. A.R. Gennaro, 2000, Lippincott Williams & Wilkins, Philadelphia, or in Encyclopedia of Pharmaceutical Technology, eds. J. Swarbrick and J. C. Boylan, 1988-1999, Marcel Dekker, New York.

Immediate release formulations for oral use include tablets or capsules containing the active ingredient(s) in a mixture with non-toxic pharmaceutically acceptable excipients. These excipients maybe, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., sucrose, glucose, mannitol, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone, or polyethylene glycol); and lubricating agents, glidants, and antiadhesives (e.g., magnesium stearate, zinc stearate, stearic acid, silicas, hydrogenated vegetable oils, or talc). Other pharmaceutically acceptable excipients can be colorants, flavoring agents, plasticizers, humectants, buffering agents, and the like are found, for example, in The Handbook of Pharmaceutical Excipients, third edition, edited by Authur H. Kibbe, Americal Pharmaceutical Association Washington DC.

Dissolution or diffusion controlled release can be achieved by appropriate coating of a tablet, capsule, pellet, or granulate formulation of compounds, or by incorporating the compound into an appropriate matrix. A controlled release coating may include one or more of the coating substances mentioned above and/or, e.g., shellac, beeswax, glycowax, castor wax, carnauba wax, stearyl alcohol, glyceryl monostearate, glyceryl distearate, glycerol palmitostearate, ethylcellulose, acrylic resins, dl-polylactic acid, cellulose acetate butyrate, polyvinyl chloride, polyvinyl acetate, vinyl pyrrolidone, polyethylene, polymethacrylate, methylmethacrylate, 2-hydroxymethacrylate, methacrylate hydrogels, 1,3 butylene glycol, ethylene glycol methacrylate, and/or polyethylene glycols. In a controlled release matrix formulation, the matrix material may also include, e.g., hydrated methylcellulose, carnauba wax and stearyl alcohol, carbopol 934, silicone, glyceryl tristearate, methyl acrylate-methyl methacrylate, polyvinyl chloride, polyethylene, and/or halogenated fluorocarbon.

A controlled release composition may also be in the form of a buoyant tablet or capsule (i.e., a tablet or capsule that, upon oral administration, floats on top of the gastric content for a certain period of time). A buoyant tablet formulation of the compound(s) can be prepared by granulating a mixture of the antibiotic with excipients and 20-75% w/w of hydrocolloids, such as hydroxyethylcellulose, hydroxypropylcellulose, or hydroxypropylmethylcellulose. The obtained granules can then be compressed into tablets. On contact with the gastric juice, the tablet forms a substantially water-impermeable gel barrier around its surface. This gel barrier takes part in maintaining a density of less than one, thereby allowing the tablet to remain buoyant in the gastric juice. Other useful controlled release compositions are known in the art (see, for example, U.S. Patent Nos. 4,946,685 and 6,261,601).

A modified release composition may be comprised of a compression-coated core whose geometric configuration controls the release profile of the encapsulated antibiotic. By varying the geometry of the core, the profile of the antibiotic release can be adjusted to follow zero order, first order or a combination of these orders. The system can also be designed to deliver more beneficial agents at the same time, each having a different release profile (see, for example U.S. Patent Nos. 4,111,202 and 3,279,995).

Formulations that target Compound I release to particular regions of the intestinal tract can also be prepared. Compound I can be encapsulated in an enteric coating that prevents release degradation and release from occurring in the stomach, but dissolves readily in the mildly acidic or neutral pH environment of the small intestine. A formulation targeted for release of antibiotic to the colon, utilizing technologies such as time-dependent, pH-dependent, or enzymatic erosion of polymer matrix or coating can also be used.

Alternatively, a multilayer formulation having different release characteristics between the layers can be prepared. These formulations can result in the antibiotic being released in different regions of the intestinal tract.

A multilayer formulation of this type may be particularly useful for maintaining a more constant antibiotic concentration throughout the length of the intestinal tract.

In one aspect of this embodiment, the protective layer is comprised of one or more components, which includes an immediate release layer and a modifying layer. The modifying layer is preferably comprised of a semi water-permeable polymer. Applicants have surprisingly found that a semi-permeable polymer coating used in combination with an immediate release layer coating provided a delayed pulsed release antibiotic delivery profile when layered over the enteric coating.

Thus, in this embodiment, the protective layer comprises a semi-permeable polymer and an immediate release coating layer. In a preferred embodiment, the modifying layer comprises a first layer of a semi-permeable polymer which is adjacent to the enteric coating layer and a second coating layer over the semi-permeable polymer coating layer comprising an immediate release polymer coating layer.

In one aspect of this embodiment, a semi-permeable polymer, which may comprise a low water-permeable pH-insensitive polymer, is layered onto the outer surface of the enteric layer, in order to obtain prolonged delayed release time. This semi-permeable polymer coating controls the erosion of the pH-sensitive enteric polymer in an alkaline pH environment in which a pH-sensitive polymer will dissolve rapidly. Another pH-sensitive layer may be applied onto the surface of a low water-permeability layer to further delay the release time.

In a still further aspect of the invention, in addition to a protective layer, the composition comprises an acid which is incorporated into the pharmaceutical active layer or coated onto the surface of the active layer to reduce the pH value of the environment around the enteric polymer layer. The acid layer may also be applied on the outer layer of the pH-sensitive enteric polymer layer, followed by a layer of low water-permeability polymer. The release of the active layer thus may be delayed and the dissolution rate may be increased in an alkaline environment.

In a further embodiment, the protective coating may be used both over the antibiotic and over the enteric coating.

The targeted delivery properties of the Compound I containing formulation may be modified by other means. For example, the antibiotic may be complexed by inclusion, ionic association, hydrogen bonding, hydrophobic bonding, or covalent bonding. In addition polymers or complexes susceptible to enzymatic or microbial lysis may also be used as a means to deliver drug.

Microsphere encapsulation of Compound I is another useful pharmaceutical formulation for targeted antibiotic release. The antibiotic-containing microspheres can be used alone for antibiotic delivery, or as one component of a two-stage release formulation. Suitable staged release formulations may consist of acid stable microspheres, encapsulating Compound I to be released later in the lower intestinal tract admixed with an immediate release formulation to deliver antibiotic to the stomach and upper duodenum.

Microspheres can be made by any appropriate method, or from any pharmaceutically acceptable material. Particularly useful are proteinoid microspheres (see, for example, U.S. Patent Nos. 5,601,846, or 5,792,451) and PLGA-containing microspheres (see, for example, U.S. Patent Nos. 6,235,224 or 5,672,659). Other polymers commonly used in the formation of microspheres include, for example, poly-ε-caprolactone, poly(e∼caprolactone-Co-DL-lactic acid), poly(DL-lactic acid), poly(DL-lactic acid-Co-glycolic acid) and poly(s-caprolactone Co-glycolic acid) (see, for example, Pitt et al., J. Pharm. Sci., 68:1534,1979). Microspheres can be made by procedures well known in the art including spray drying, coacervation, and emulsification (see for example Davis et al. Microsphere and Drug Therapy, 1984, Elsevier; Benoit et al. Biodegradable Microspheres: Advances in Production Technologies, Chapter 3, ed. Benita, S, 1996, Dekker, New York; Microencapsulation and Related Drug Processes, Ed. Deasy, 1984, Dekker, New York; U.S. Patent No. 6,365,187).

Powders, dispersible powders, or granules suitable for preparation of aqueous solutions or suspensions of Compound I by addition of water are convenient dosage forms for oral administration. Formulation as a suspension provides the active ingredient in a mixture with a dispersing or wetting agent, suspending agent, and one or more preservatives. Suitable dispersing or wetting agents are, for example, naturally-occurring phosphatides (e.g., lecithin or condensation products of ethylene oxide with a fatty acid, a long chain aliphatic alcohol, or a partial ester derived from fatty acids) and a hexitol or a hexitol anhydride (e.g., polyoxyethylene stearate, polyoxyethylene sorbitol monooleate, polyoxyethylene sorbitan monooleate, and the like). Suitable suspending agents are, for example, sodium carboxymethylcellulose, methylcellulose, sodium alginate, and the like.

### Brief Description of the Tables

Table 1. Tiacumicin A-F
Table 2. is a summary of activity of Compound I against laboratory strains from American Type Culture Collection (ATCC)
Table 3. is a summary of activity of Compound I against clinical isolates of bacteria
Table 4. is a summary of activity of Compound I and vancomycin against 207 clinical isolates of *C*. *difficile*
Table 5. is a summary of activity of Compound I and vancomycin against 102 clinical isolates of *Clostridium* species
Table 6. is a summary of activity of Compound I and vancomycin against 322 clinical isolates from gastrointestinal tract
Table 7. Geometric mean, MIC ranges, MIC₅₀, and MIC₉₀ values for Compound I against 110 *C. difficile* clinical isolates, vancomycin, and metronidazole, in µg/mL.
Table 8. Raw MIC data for Compound I, vancomycin (VAN), and metronidazole (MTZ) versus 110 clinical isolates of *C. difficile,* in µg/mL.

### EXAMPLES

The invention will be further illustrated by reference to the following nonlimiting Examples.

### EXAMPLE 1

### In vitro activity of Compound I against laboratory and clinical strains of bacteria.

The activity of Compound I was tested against laboratory strains of different species of bacteria using NCCLS antimicrobial susceptibility testing guidelines. Compound I demonstrated excellent activity against *Clostridium* sp, *Micrococcus* sp. and moderate activity against *Staphylococcus* sp. Including MRSA and *Enterococcus* sp. Including VRE (Table. 2).

**Table 2. Activity of Compound I against laboratory strains from American TypeCulture Collection (ATCC)**

| **Gram negative bacteria** | **n** | **Range** | **Gram positive bacteria** | **n** | **Range** |
|---|---|---|---|---|---|
| *Acinetobacter* sp. | 2 | 1 - >32 | *Bacillus* sp. | 2 | 1 |
| *Bacteroides* sp. | 5 | >32 | *Clostridium* sp. | 4 | ≤0.015 - 0.0625 |
| *Campylobacter* sp. | 3 | 64 - >64 | *Enterococcus* sp. (incl. VRE) | 4 | 4 |
| *Citrobacter* sp. | 2 | >64 | *Lactobacillus* sp. | 3 | 1 - >32 |
| Enterobacteriaceae. | 10 | >32 | *Micrococcus* sp. | 4 | ≤0.125 |
| *Fusobacterium* sp. | 1 | >32 | Anaerobic Gram positive cocci | 4 | ≤0.06 - 1 |
| *Helicobacter* sp. | 1 | >32 | *Staphylococcus* sp. (incl. MRSA) | 6 | 1 - 16 |
| *Moraxella* sp. | 2 | 1 - 2 | *Streptococcus* sp. | 5 | 16 - 32 |
| *Neisseria* sp. | 3 | 8 - 64 | | | |

Compound I was additionally tested against anaerobic clinical isolates and against aerobic bacteria. A panel of 207 *C. difficile* clinical isolates, were shown to be very sensitive to Compound I. The compound was also active against clinical strains of *Staphylococcus* sp. and *Enterococcus* sp. These results demonstrate the narrow antimicrobial spectrum of this compound against certain pathogenic gram-positive organisms (Table 3 and 4).

**Table 3. Activity of Compound I against clinical isolates**

| Bacteria | n | Range | MIC₅₀ (µg/mL) |
|---|---|---|---|
| *Clostridium difficile* | 207 | ≤0.0009 - 0.0625 | 0.0019 |
| *Bacteroides fragilis* | 69 | >128 | >128 |
| *Prevotella* sp. | 35 | 16 -≥128 | ≥128 |
| *Eubacterium* sp. | 26 | 8 -≥128 | 32 |
| *Lactobacillus* sp. | 8 | 8-32 | Not done |
| *Propionibacterium* sp. | 16 | ≤0.031 -≥128 | 4 |
| *Enterococcus* sp. | 8 | 1 - 8 | 4 |
| *Staphylococcus* sp. | 10 | 1 - 8 | 2 |
| *Streptococcus* sp. | 10 | 8 - >64 | 16 |
| Enterobacteiaceae | 28 | >64 | >64 |
| *Pseudomonas* sp. | 15 | >64 | >64 |

**Table 4. Activity of Compound I and Vancomycin against 207 clinical isolates of C. difficile**

| Drug | Range | MIC₅₀ (µg/mL) | MIC₉₀ (µg/mL) |
|---|---|---|---|
| Compound I | ≤0.0009 - 0.0625 | 0.002 | 0.008 |
| Vancomycin | 0.0156 - 0.5 | 0.5 | 0.5 |

In another study, various clinical isolates of *Clostridium* species and over 300 clinical GI isolates were tested versus Compound I. Compound I was most active against *C. difficile, C. perfringens and C. sordellii* with the MIC₉₀ between 0.062 and 0.25 µg/mL (Table 5 and 6). Compound I was also active against *Staphylococcus* and *Enterococcus* with MIC₉₀ at 1 and 8 µg/mL, respectively.

**Table 5. Activity of Compound I and Vancomycin against clinical isolates of Clostridium specis**

| | | **Compound I** | | | **Vancomycin** | | |
|---|---|---|---|---|---|---|---|
| Bacteria | n | Range (µg/mL) | MIC₅₀ (µg/mL) | MIC₉₀ (µg/mL) | Range (µg/mL) | MIC₅₀ (µg/mL) | MIC₉₀ (µg/mL) |
| *C. bolteae* | 6 | 1 - >1024 | >1024 | >1024 | 1 - 16 | 16 | 16 |
| *C. clostridioforme* | 5 | 2->1024 | 4 | >1024 | 1 - 8 | 1 | 8 |
| *C. difficile* | 23 | 0.062 - 2 | 0.12 | 0.25 | 0.5 - 4 | 1 | 2 |
| *C. glycolicum* | 9 | 0.062 - 1 | 0.062 | 1 | 0.5 - 1 | 1 | 1 |
| *C. innocuum* | 9 | 32->1024 | >1024 | >1024 | 8 - 16 | 16 | 16 |
| *C. paraputrificum* | 10 | 0.062 - 64 | 8 | 32 | 1 - 2 | 2 | 2 |
| *C. perfringens* | 14 | 0.062 | 0.062 | 0.062 | 0.5 - 1 | 1 | 1 |
| *C. ramosum* | 10 | 16->1024 | >1024 | >1024 | 4-8 | 4 | 8 |
| *C. sordellii* | 5 | 0.062 | 0.062 | 0.062 | 1 | 1 | 1 |
| Other *Clostridium* sp. | 11 | 0.06->1024 | 32 | 1024 | 1-64 | 1 | 16 |

**Table 6. Activity of Compound I and Vancomycin against clinical gastrointestinal isolates**

| | | **OPT-80** | | | **Vancomycin** | | |
|---|---|---|---|---|---|---|---|
| Bacteria | n | Range (µg/mL) | MIC₅₀ (µg/mL) | MIC₉₀ (µg/mL) | Range (µg/mL) | MIC₅₀ (µg/mL) | MIC₉₀ (µg/mL) |
| *Bacteroides fragilis* group | 54 | >128 - >1024 | 512 | >1024 | 16 - 256 | 64 | 128 |
| *Veillonella* sp. | 10 | 16 - 128 | 32 | 128 | 128->1024 | 512 | 512 |
| Other anaerobic Gram negative rods | 51 | 0.06->1024 | 1024 | >1024 | 0.5->1024 | 512 | >1024 |
| Non-spore forming Gram positive rods | 64 | 0.06->1024 | 1 | 32 | 0.5->1024 | 128 | >1024 |
| Anaerobic Gram positive cocci | 49 | 0.06-1024 | 0.5 | 2 | 0.5->1024 | 1 | 8 |
| *Streptococcus milleri* group | 14 | 16-64 | 32 | 32 | 1 | 1 | 1 |
| *Streptococcus* sp. | 9 | 16 - 128 | 32 | 128 | 0.5 - 1 | 0.5 | 1 |
| *Eschericia coli* | 10 | >256 | >256 | >256 | 256->1024 | 512 | >1024 |
| *Enterobacter* sp. | 20 | >256 | >256 | >256 | 256->1024 | >1024 | >1024 |
| *Klebsiella* sp. | 10 | >256 | >256 | >256 | >1024 | >1024 | >1024 |
| *Proteus mirabilis* | 10 | >256 | >256 | >256 | >1024 | >1024 | >1024 |
| *Pseudomonas aeruginosa* | 10 | 64->256 | >256 | >256 | 1024->1024 | >1024 | >1024 |
| *Enterococcus* sp. | 22 | 0.5-16 | 8 | 8 | 0.5-4 | 1 | 4 |
| *Staphylococcus* sp. | 19 | 0.25-2 | 0.5 | 1 | 1-4 | 2 | 4 |

### EXAMPLE 2

### Comparative Efficacy of Compound I, Metronidazole, and Vancomycin in the 5 hamster model of Clostridium difficile Associated Diarrhea.

To evaluate the *in vivo* efficacy of Compound I in the treatment of *Clostridium difficile*-associated colitis, Compound I was tested in a hamster model of clindamycin-induced colitis in comparison with both vancomycin and metronidazole. Animals were treated with two oral doses of clindamycin at 100 mg/kg. Three days after the second dose of clindamycin, they were inoculated with toxigenic *C. difficile* spores. Eight hours after infection the animals received oral Compound I, vancomycin or metronidazole for 7 days. Animals were observed daily for the presence or absence of diarrhea. Necropsies were performed on some animals that died during the experiment, and cecal contents were assayed for *C. difficile* toxin A. Hamsters were monitored for 20 days, and the cumulative mortality during this period was recorded (Fig. 1). All three tested antibiotics protected the animals from infection, which-in the absence of treatment-was otherwise uniformly fatal between days two and six post-infection. The ED₅₀ for Compound I was below 0.3 mg/kg. Treatment with Compound I at concentrations of 0.8 and 2.5 mg/kg was as effective as treatment with vancomycin (5 mg/kg) or metronidazole (100 mg/kg).

### EXAMPLE 3

### Oral Administration of Compound I to Humans.

Tolerability and pharmacokinetics of Compound I following single dose administration was investigated in 16 healthy volunteer subjects. The clinical trial was a single dose, double blinded, randomized, placebo-controlled, dose escalation study.

Compound I was administered orally after a morning breakfast to volunteer 10 subjects. Plasma, urine, and fecal concentrations of Compound I were determined for pharmacokinetic evaluation.

After oral administration, little Compound I was detected in the blood; concentrations were near the lower limit of quantitation (LLOQ = 5 ng/mL). Only one subject in the 450 mg dose group had plasma concentrations that were detectable as late as 8 hours. The highest plasma level observed was 37.8 ng/mL (in the highest dose, 450 mg group).

The fecal recovery of unchanged Compound I as a percent dose of administered was about 20% in the 200 and 300 mg dosing groups with the mean values for the corresponding peak fecal concentration were 157 and 248 µg/g, respectively.

### EXAMPLE 4

### In Vitro Activity of Compound I

The *in vitro* efficacy of Compound I, metronidazole, and vancomycin were assessed versus 110 genetically distinct clinical isolates of *C*. *difficile* via agar dilution. The MIC data are presented in Tables 7 and 8.

**Table 7. Geometric mean, MIC ranges, MIC₅₀, and MIC₉₀ values for Compound I against 110 C. difficile clinical isolates, vancomycin, and metronidazole, in µg/mL.**

| | **Range** | **Geometric Mean** | **MIC₅₀** | **MIC₉₀** |
|---|---|---|---|---|
| **Compound I** | 0.015 - 0.25 | 0.08 | 0.125 | 0.125 |
| **Metronidazole** | 0.025 - 0.5 | 0.15 | 0.125 | 0.25 |
| **Vancomycin** | 0.06 - 4 | 0.8 | 1 | 1 |

**Table 8. Raw MIC data for Compound I, vancomycin (VAN), and metronidazole (MTZ) versus 110 clinical isolates of C. difficile, in µg/mL.**

| **ORG ID** | **Compound I** | **MTZ** | **VAN** | **ORG ID** | **Compound I** | **MTZ** | **VAN** |
|---|---|---|---|---|---|---|---|
| **A1 1535** | 0.125 | 0.25 | 1 | **CO1 4652** | 0.25 | 0.125 | 1 |
| **B1 832** | 0.06 | 0.125 | 1 | **CP1 5491** | 0.125 | 0.25 | 1 |
| **D1 1360** | 0.03 | 0.25 | 1 | **61 5930** | 0.03 | 0.25 | 1 |
| **E1 816** | 0.06 | 0.125 | 1 | **63 6029** | 0.25 | 0.25 | 0.06 |
| **F1 1015** | 0.125 | 0.125 | 1 | **64 5940** | 0.125 | 0.25 | 1 |
| **G1 1077** | 0.125 | 0.125 | 1 | **65 5967** | 0.06 | 0.25 | 0.5 |
| **I1 1389** | 0.125 | 0.125 | 1 | **66 6366** | 0.015 | 0.125 | 0.5 |
| **J1 5971** | 0.06 | 0.25 | 1 | **67 6367** | 0.125 | 0.25 | 1 |
| **J7 4224** | 0.03 | 0.125 | 1 | **68 6368** | 0.03 | 0.125 | 0.06 |
| **J9 4478** | 0.06 | 0.125 | 1 | **69 6370** | 0.25 | 0.25 | 0.5 |
| **K1 4305** | 0.125 | 0.25 | 0.5 | **70 6376** | 0.125 | 0.25 | 2 |
| **K14 5780** | 0.125 | 0.125 | 1 | **71 6379** | 0.125 | 0.25 | 1 |
| **L1 1423** | 0.125 | 0.125 | 0.5 | **72 6380** | 0.125 | 0.25 | 2 |
| **N1 471** | 0.125 | 0.125 | 0.5 | **73 6382** | 0.25 | 0.25 | 1 |
| **O1 1861** | 0.06 | 0.125 | 1 | **75 6388** | 0.125 | 0.125 | 0.5 |
| **R1 397** | 0.125 | 0.125 | 1 | **76 6389** | 0.125 | 0.25 | 0.5 |
| **R6 6015** | 0.015 | 0.25 | 2 | **77 6390** | 0.06 | 0.125 | 1 |
| **V1 1521** | 0.125 | 0.125 | 0.5 | **78 6392** | 0.015 | 0.03 | 0.5 |
| **W1 3931** | 0.125 | 0.5 | 1 | **80 6327** | 0.125 | 0.125 | 0.5 |
| **X1 1890** | 0.125 | 0.125 | 1 | **81 6328** | 0.125 | 0.125 | 0.5 |
| **Y1 5639** | 0.06 | 0.125 | 0.5 | **82 6329** | 0.06 | 0.03 | 0.5 |
| **Y2 1459** | 0.06 | 0.125 | 1 | **83 6330** | 0.06 | 0.125 | 0.5 |
| **Z1 3036** | 0.03 | 0.125 | 1 | **84 6331** | 0.125 | 0.25 | 0.5 |
| **AA2 4380** | 0.015 | 0.125 | 1 | **85 6332** | 0.06 | 0.125 | 1 |
| **AB2 1725** | 0.06 | 0.125 | 1 | **86 6333** | 0.03 | 0.125 | 0.5 |
| **AC1 1546** | 0.06 | 0.125 | 1 | **87 6334** | 0.125 | 0.125 | 0.5 |
| **AF1 1808** | 0.125 | 0.125 | 0.5 | **88 6335** | 0.125 | 0.25 | 0.5 |
| **AG1 3044** | 0.125 | 0.125 | 1 | **89 6336** | 0.25 | 0.5 | 1 |
| **AH1 3430** | 0.125 | 0.25 | 0.5 | **90 6338** | 0.125 | 0.125 | 1 |
| **AJ1 1557** | 0.06 | 0.125 | 1 | **91 6339** | 0.125 | 0.125 | 1 |
| **AL1 1753** | 0.06 | 0.125 | 0.5 | **93 6341** | 0.125 | 0.125 | 1 |
| **AN1 464** | 0.125 | 0.125 | 0.5 | **94 6343** | 0.015 | 0.06 | 0.5 |
| **AO1 287** | 0.125 | 0.125 | 1 | **95 6347** | 0.125 | 0.125 | 1 |
| **AS1 4099** | 0.125 | 0.125 | 1 | **96 6348** | 0.06 | 0.125 | 0.5 |
| **AT1 1216** | 0.125 | 0.125 | 1 | **97 6349** | 0.25 | 0.125 | 1 |
| **AV1 941** | 0.25 | 0.125 | 0.5 | **98 6350** | 0.125 | 0.5 | 1 |
| **CJ1 893** | 0.125 | 0.025 | 1 | **101 6354** | 0.015 | 0.06 | 1 |
| **AW1 4501** | 0.125 | 0.125 | 1 | **102 6355** | 0.016 | 0.125 | 1 |
| **BE1 4307** | 0.125 | 0.25 | 1 | **103 6068** | 0.06 | 0.125 | 1 |
| **BH1 4506** | 0.06 | 0.06 | 0.5 | **104 6060** | 0.03 | 0.25 | 1 |
| **BI1 1675** | 0.125 | 0.125 | 1 | **105 6071** | 0.03 | 0.125 | 0.5 |
| **BK1 4291** | 0.125 | 0.125 | 0.5 | **106 6078** | 0.03 | 0.25 | 0.5 |
| **BL1 716** | 0.125 | 0.125 | 1 | **107 6079** | 0.06 | 0.125 | 0.5 |
| **BM1 1453** | 0.06 | 0.125 | 1 | **109 6274** | 0.015 | 0.125 | 1 |
| **BN1 1322** | 0.125 | 0.25 | 1 | **111 6279** | 0.03 | 0.125 | 1 |
| **BR1 1321** | 0.06 | 0.125 | 1 | **112 6280** | 0.06 | 0.125 | 0.5 |
| **BT1 706** | 0.06 | 0.125 | 1 | **113 6304** | 0.06 | 0.125 | 1 |
| **BV1 1183** | 0.125 | 0.25 | 1 | **114 386** | 0.06 | 0.125 | 4 |
| **BW1 3130** | 0.125 | 0.125 | 1 | **115 5985** | 0.015 | 0.25 | 2 |
| **BX1 4271** | 0.125 | 0.25 | 1 | **116 5702** | 0.06 | 0.125 | 1 |
| **CN1 667** | 0.25 | 0.25 | 1 | **117 6026** | 0.06 | 0.125 | 2 |
| **CB1 1584** | 0.25 | 0.125 | 1 | **120 6057** | 0.03 | 0.25 | 1 |
| **CF1 5922** | 0.125 | 0.125 | 1 | **121 6072** | 0.06 | 0.25 | 0.5 |
| **CG1 1566** | 0.125 | 0.125 | 1 | **122 6111** | 0.25 | 0.25 | 0.5 |
| **CL1 3851** | 0.25 | 0.125 | 1 | **100 6353** | 0.125 | 0.25 | 1 |

### Other Embodiments

All references discussed above are herein incorporated by reference in their entirety for all purposes. While this invention has been particularly shown and described with references to preferred embodiments thereof, it will be understood by those skilled in the art that various changes in form and details may be made therein without departing from the spirit and scope of the invention as defined by the appended claims.

## Claims

1. A compound having the formula: 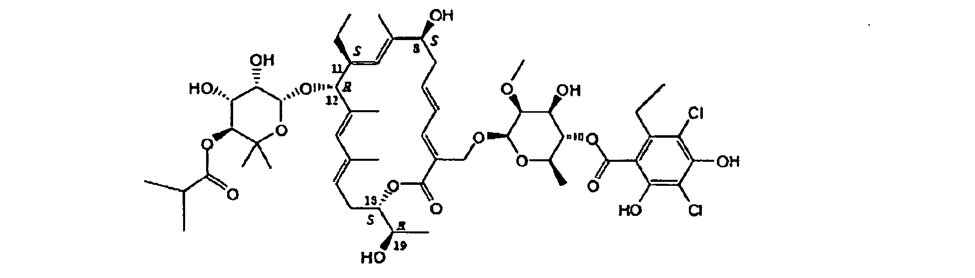 for use in treating a disease in a patient caused by the presence of *Clostridium difficile.*

2. The compound of claim 1 for use according to claim 1, wherein the patient is a mammal.

3. The compound of claim 1 for use according to claim 1, wherein the patient is a human.

4. The compound of claim 1 for use according to claim 1, wherein the disease is associated with the use of antibiotics

5. The compound of claim 1 for use according to claim 1, wherein the disease is associated with the use of chemotherapy.

6. The compound of claim 1 for use according to claim 1, wherein the disease is associated with the use of antiviral therapy.

7. The compound of Claim 4 for use according to claim 1, wherein the disease is antibiotic-associated diarrhea.

8. The compound of any one of Claims 1 to 6 for use according to claim 1, wherein the disease is colitis.

9. The compound of any one of Claims 1 to 6 for use according to claim 1, wherein the disease is pseudomembranous colitis.

10. The compound of any of the claims 1 to 9 for use according to claim 1, wherein the compound is for administration in an amount of about 10 mg to about 1g.

11. The compound of claim 10 for use according to claim 1, wherein the compound is for administration in an amount of about 50 mg to about 400 mg once or twice daily.

12. The compound of any of claims 1 to 10 for use according to claim 1, wherein the compound is for administration from one to four times daily.

13. The compound of any of claims 1 to 12 for use according to claim 1, wherein the compound is for administration to the patient orally.

14. The compound of any of claims 1 to 10 for use according to claim 1, wherein the compound is for administration from one to four times daily for three to fifteen days.

15. The compound of claim 1 for use according to claim 1, wherein the compound is for use in treating *Clostridium difficile* associated diarrhea in a patient by oral administration.

## Patentansprüche

1. Eine Verbindung der Formel: 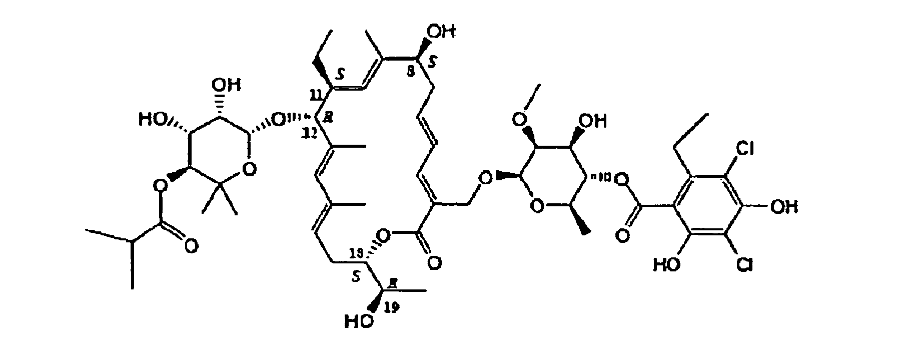 zur Verwendung bei der Behandlung einer durch die Gegenwart von *Clostridium difficile* hervorgerufenen Erkrankung bei einem Patienten.

2. Die Verbindung nach Anspruch 1 zur Verwendung gemäß Anspruch 1, wobei der Patient ein Säuger ist.

3. Die Verbindung nach Anspruch 1 zur Verwendung gemäß Anspruch 1, wobei der Patient ein Mensch ist.

4. Die Verbindung nach Anspruch 1 zur Verwendung gemäß Anspruch 1, wobei die Erkrankung mit der Anwendung von Antibiotika assoziiert ist.

5. Die Verbindung nach Anspruch 1 zur Verwendung gemäß Anspruch 1, wobei die Erkrankung mit der Anwendung von Chemotherapie assoziiert ist.

6. Die Verbindung nach Anspruch 1 zur Verwendung gemäß Anspruch 1, wobei die Erkrankung mit der Anwendung einer antiviralen Therapie assoziiert ist.

7. Die Verbindung nach Anspruch 4 zur Verwendung gemäß Anspruch 1, wobei die Erkrankung Antibiotika-assoziierte Diarrhö ist.

8. Die Verbindung nach einem der Ansprüche 1 bis 6 zur Verwendung gemäß Anspruch 1, wobei die Erkrankung Colitis ist.

9. Die Verbindung nach einem der Ansprüche 1 bis 6 zur Verwendung gemäß Anspruch 1, wobei die Erkrankung pseudomembranöse Colitis ist.

10. Die Verbindung nach einem der Ansprüche 1 bis 9 zur Verwendung gemäß Anspruch 1, wobei die Verbindung zur Verabreichung in einer Menge von etwa 10 mg bis etwa 1 g vorgesehen ist.

11. Die Verbindung nach Anspruch 10 zur Verwendung gemäß Anspruch 1, wobei die Verbindung zur Verabreichung in einer Menge von etwa 50 mg bis etwa 400 mg einmal oder zweimal täglich vorgesehen ist.

12. Die Verbindung nach einem der Ansprüche 1 bis 10 zur Verwendung gemäß Anspruch 1, wobei die Verbindung zur Verabreichung einmal bis viermal täglich vorgesehen ist.

13. Die Verbindung nach einem der Ansprüche 1 bis 12 zur Verwendung gemäß Anspruch 1, wobei die Verbindung zur oralen Verabreichung an den Patienten vorgesehen ist.

14. Die Verbindung nach einem der Ansprüche 1 bis 10 zur Verwendung gemäß Anspruch 1, wobei die Verbindung vorgesehen ist zur Verabreichung einmal bis viermal täglich, drei bis fünfzehn Tage lang.

15. Die Verbindung nach Anspruch 1 zur Verwendung gemäß Anspruch 1, wobei die Verbindung zur Verwendung bei der Behandlung von *Clostridiumdifficile*-assoziierter Diarrhö bei einem Patienten durch orale Verabreichung vorgesehen ist.

## Revendications

1. Composé ayant la formule: 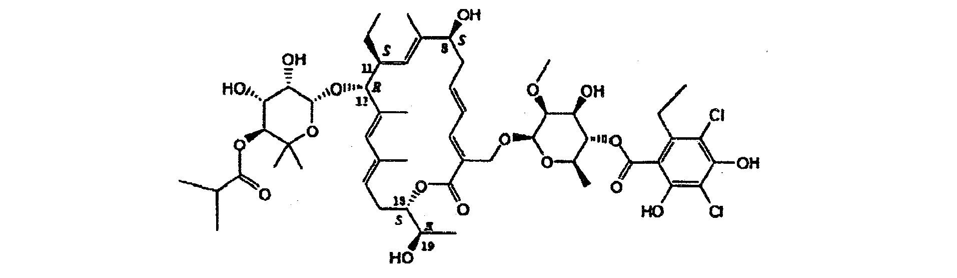 pour l'utilisation dans le traitement d'une maladie chez un patient provoquée par la présence de *Clostridium difficile.*

2. Composé selon la revendication 1 pour l'utilisation selon la revendication 1, dans lequel le patient est un mammifère.

3. Composé selon la revendication 1 pour l'utilisation selon la revendication 1, dans lequel le patient est un humain.

4. Composé selon la revendication 1 pour l'utilisation selon la revendication 1, dans lequel la maladie est associée à l'utilisation d'antibiotiques.

5. Composé selon la revendication 1 pour l'utilisation selon la revendication 1, dans lequel la maladie est associée à l'utilisation d'une chimiothérapie.

6. Composé selon la revendication 1 pour l'utilisation selon la revendication 1, dans lequel la maladie est associée à l'utilisation d'une thérapie antivirale.

7. Composé selon la revendication 4 pour l'utilisation selon la revendication 1, dans lequel la maladie est une diarrhée associée à un antibiotique.

8. Composé selon l'une quelconque des revendications 1 à 6 pour l'utilisation selon la revendication 1, dans lequel la maladie est une colite.

9. Composé selon l'une quelconque des revendications 1 à 6 pour l'utilisation selon la revendication 1, dans lequel la maladie est une colite pseudomembraneuse.

10. Composé selon l'une quelconque des revendications 1 à 9 pour l'utilisation selon la revendication 1, lequel composé est pour l'administration en une quantité d'environ 10mg à environ 1g.

11. Composé selon la revendication 10 pour l'utilisation selon la revendication 1, lequel composé est pour l'administration en une quantité d'environ 50mg à environ 400mg une ou deux fois par jour.

12. Composé selon l'une quelconque des revendications 1 à 10 pour l'utilisation selon la revendication 1, lequel composé est pour l'administration d'une à quatre fois par jour.

13. Composé selon l'une quelconque des revendications 1 à 12 pour l'utilisation selon la revendication 1, lequel composé est pour l'administration au patient oralement.

14. Composé selon l'une quelconque des revendications 1 à 10 pour l'utilisation selon la revendication 1, lequel composé est pour l'administration d'une à quatre fois par jour pendant trois à quinze jours.

15. Composé selon la revendication 1 pour l'utilisation selon la revendication 1, lequel composé est pour l'utilisation dans le traitement d'une diarrhée associée à *Clostridium difficile* chez un patient par administration orale.
